(19) Europäisches Patentamt
European Patent Office
Office européen des brevets

(11) Publication number: **0 188 039**
**B1**

(12) # EUROPEAN PATENT SPECIFICATION

(45) Date of publication of patent specification: **09.08.89**

(51) Int. Cl.$^4$: **C 07 D 251/18**

(21) Application number: **85300183.2**

(22) Date of filing: **10.01.85**

(54) **Process for synthesis of 2-vinyl-4,6-diamino-S-triazine.**

(43) Date of publication of application:
**23.07.86 Bulletin 86/30**

(45) Publication of the grant of the patent:
**09.08.89 Bulletin 89/32**

(84) Designated Contracting States:
**BE CH DE FR GB IT LI**

(56) References cited:
**DE-A-2 026 400**
**DE-A-2 135 881**
**FR-A-1 563 255**

(73) Proprietor: **SHIKOKU CHEMICALS CORPORATION**
**147-1-Minato-machi Marugame-shi**
**Kagawa-ken (JP)**

(72) Inventor: **Sawa, Natsuo**
**27, 5-chome Wakaba-cho Tadotsu-cho**
**Nakatado-gun Kagawa-ken (JP)**
Inventor: **Masuda, Takeshi**
**8-15, 2-chome, Kita-Hirayama-cho**
**Marugame-shi Kagawa-ken (JP)**

(74) Representative: **Goldin, Douglas Michael et al**
**J.A. KEMP & CO. 14, South Square Gray's Inn**
**London WC1R 5EU (GB)**

**EP 0 188 039 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the synthesis of 2-vinyl-4,6-diamino-S-triazine (hereinafter referred to as "VT"). More particularly, the present invention provides a process for preparing VT in large quantities at low costs.

VT is a compound which is valuable as a comonomer, and it is known that if a diamino-S-triazine is introduced into side chains of a polymer, the softening point and glass transition point of the polymer are greatly elevated as compared with those of the unintroduced polymer, and the specific gravity is increased and the solubility is prominently changed (see, for example, Nadao and Kakurai: Collection of Polymer Theses, *32*, 308 (1975) and T. Seo, K. Abe, H. Honma and T. Kakurai: Polym. Prepn., *20*, 661 (1979)).

Several processes as described below are known for the synthesis of VT. Namely, there can be mentioned a process in which biguanide is reacted with acrylic acid chloride (C. G. Overberger et al; J.A.C.S., *80*, 988 (1958)), a process in which dicyandiamide is reacted with β-dimethylaminopropionitrile (French Patent No. 1,563,255 (1967) to Hoechst AG), a prcoess in which 1,2-di(4,6-diamino-S-triazinyl-(2))-cyclobutane is heated at 320°C under reduced pressure JP—A—35068/71 to Asahi Kasei), and a process in which 2-β-methoxyethyl-4,6-diamino-S-triazine is heated at 350°C in a nitrogen current (Suddeutsche Kalkstickstoff Werke A. G.: DE—A—2,135,881 (1973)).

However, these known processes are defective in that the starting materials are expensive or the reaction procedures are complicated. Accordingly, none of these known processes are suitable for industrial working.

It is therefore a primary object of the present invention to provide a process for synthesizing VT in large quantities at low costs, which is suitable for industrial working.

More specifically, in accordance with the present invention, there is provided a process for the synthesis of 2-vinyl-4,6-diamino-S-triazine having the following structural formula:

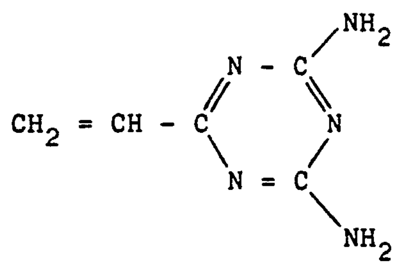

which comprises heating an imidazolyl-S-triazine compound represented by the following general formula:

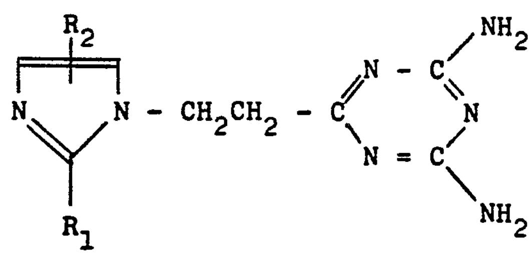

wherein $R_1$ stands for a hydrogen atom, a methyl group or an ethyl group, and $R_2$ stands for a hydrogen atom or a methyl group, or the isocyanuric acid-addition product thereof under reduced pressure in the presence of a polymerization inhibitor.

The process of the present invention can be expressed by the following reaction formula:

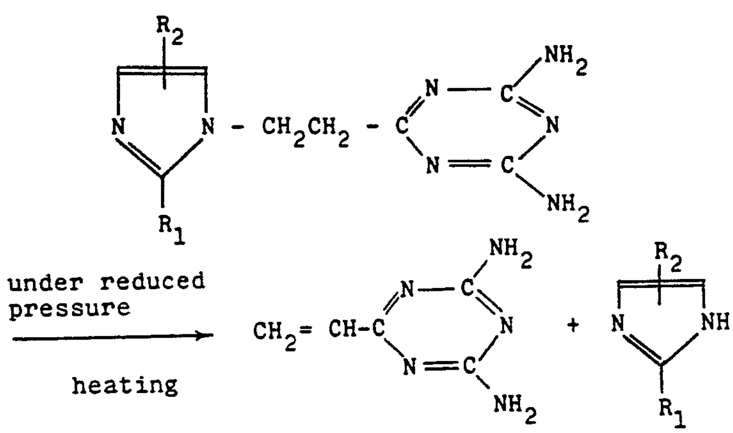

wherein $R_1$ and $R_2$ are as defined above,

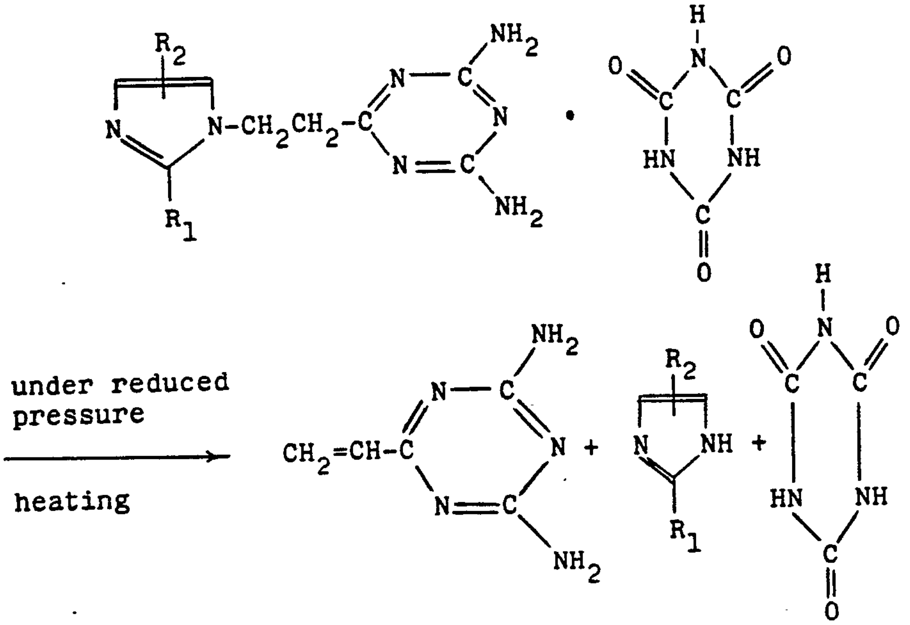


wherein $R_1$ and $R_2$ are as defined above.

The starting imdiazolyl-S-triazine compound used in the process of the present invention is easily obtained from acrylonitrile, an imidazole compound and dicyandiamide according to the process disclosed in JP—A—36391/72.

The isocyanuric acid-addition product of the imidazolyl-S-triazine compound is easily obtained by reacting an imidazolyl-S-triazine compound with isocyanuric acid according to the process disclosed in US—A—4,205,156.

The yield in the reaction of forming the imidazolyl-S-triazine compound or its isocynuric acid-addition product from the imidazole compound is very good, and the operation procedures for forming VT from this starting substance are very simple and the yield is very good, which will readily be understood from Examples given hereinafter. Accordingly, the reaction of the present invention is very suitable for working on an industrial scale.

Embodiments of the reaction of the present invention will now be described in detail.

The starting imidazolyl-S-triazine compound is conveniently heated at a temperature of 180 to 320°C under a reduced pressure of about 0.665 to about 2.66 kPa (about 5 to about 20 mmHg) for an appopriate time in the presence of an appropriate polymerization inhibitor in an appropriate reaction vessel in which a reduced pressure as described above can be maintained. By this heating, VT and the imidazole compound are sublimated to adhere to the wall of the upper portion of the reaction vessel. It is preferred that the wall of the upper portion of the reaction vessel be air-cooled or water-cooled.

The adhering reaction product is collected and washed with water to remove the imidazole compound, and the residue is recrystallized from water to obtain intended VT.

When the isocyanuric acid-addition adduct of the imidazolyl-S-triazine compound is used, the adduct is conveniently heated at 180 to 320°C under a reduced pressure of about 0.665 to about 2.66 kPa (about 5 to about 20 mmHg for an appropriate time in the presence of an appropriate polymerization inhibitor in an appropriate reaction vessel in which a reduced pressure as described above can be maintained. VT and the imidazole compound are sublimated to adhere to the wall of the upper portion of the reaction vessel. It is preferred that the wall of the upper portion of the reaction vessel be air-cooled or water-cooled.

A higher degree of the pressure reduction is preferred, but about 0.665 kPa (about 5 mmHg) is sufficient. The reaction time is within one hour.

The adhering reaction product is washed with water to remove the imidazole compound and isocyanuric acid. The residue is recrystallized from water to obtain intended VT.

As typical examples of the polymerization inhibitor to be used in the present invention, there can be mentioned sodium sulfide, potassium sulfide, hydroquinone, copper sulfate and β-naphthylamine. Among these compounds, sodium sulfide gives a highest polymerization-inhibiting effect.

In carrying out the process of the present invention, a powdery or granular heat-conducting medium may be present in the reaction system so as to increase the heat conductivity in the reaction system. As typical instances of the powdery or granular heat-conducting medium to be used in the present invention, there can be mentioned quartz sand, sea sand, river sand, glass powder, silica powder, alumina powder, iron powder, copper powder, brass powder, bronze powder, aluminum powder and zinc powder. It is preferred that the size of the powdery or granular heat-conducting medium be in the range of 32 to 7 mesh

according to the Tyler standard. It also is preferred that the powdery or granular heat-conducting medium be mixed with the starting substance so that the amount of the medium is larger than the amount of the starting substance based on the weight, and the reaction be then carried out.

The properties of VT are now described.

Melting Point: 239 to 241°C

Solubility Characteristics: Soluble in hot water and difficulty soluble in hot methanol, hot ethanol and hot acetone.

Basicity: Substantially neutral.

Polymerizability: A polymer insoluble in hot water is obtained when VT is dissolved in hot water and azobisisobutyronitrile is added to the solution.

TLC (alumina, silica and EtOH): Rf = 0.0

$\nu^{KBr}_{cm^{-1}}$: 3340, 3170, 1680 (fourth absorption), 1655 (second absorption), 1550 (first absorption), 1460 (fifth absorption), 1425 (third absorption), 1370, 1265, 1130. 985, 960, 835 (sixth absorption)

NMR ($d_6$-DMSO), γ: 6.76 (multiplet, 4H), 6.35-6.45 (triplet, 2H), 5.59—5.72 (quadruplet, 1H)

Elementary Analysis Values: C = 44.28%, H = 5.07%, N = 50.52%

The present invention will now be described in detail with reference to the following Examples that by no means limit the scope of the invention.

Example 1

In 7 ml of water were dissolved 20.5. g (0.1 mole) of a crystal of 2-(β-imidazolyl-1')-ethyl-4,6-diamino-S-triazone and 2.2 g (0.008 mole) of $Na_2S \cdot 9H_2O$, and the aqueous solution was sufficiently mixed and dried and solidified under reduced pressure to obtain a starting material. This operation was performed so that the polymerisation inhibitor was uniformly stuck to the crystal of the imidazolyl-S-triazine compound.

The so-obtained starting material and 25 g of quartz sand (having a size of 16 Tyler mesh) were charged in a Claisen flask having a side arm and an inner capacity of about 100 ml, and the bottom portion of the flask was wrapped with a hood of an electric heater and heating was carried out under a reduced pressure of 0.399 kPa (3 mmHg). Thus, the inner temperature of the flask was maintained at 250°C for 0.5 hour. After natural cooling, the sublimation product adhering to the inner wall of the upper portion and side arm of the flask was collected, and 36 ml of water was added to the collected sublimation product and the mixture was stirred at room temperature for a while. The insoluble substance (crude VT) was recovered by filtration and recrystallised from 100 mm of water to obtain 8.5 g (the yield being 62%) of intended VT characterized by a melting point of 239 to 241°C and a TLC value (silica and EtOH, coloration with $I_2$) of Rf = 0.00 to 0.01. The filtrate was distilled under reduced pressure to 4.9 g of imidazole (the recovery ratio was 72%).

Example 2

In 7 ml of water were dissolved 21.9 g (0.1 mole) of a crystal of 2-(β-(2'-methylimidazolyl-1'))-ethyl-4,6-diamino-S-triazine and 2.2 g (0.008 mole) of $Na_2S \cdot 9H_2O$, and the aqueous solution was sufficiently mixed, and dried and solidified under reduced pressure to obtain a starting material. This operation was performed so that the polymerization inhibitor was uniformly stuck to the crystal of the imidazolyl-S-triazine compound. The starting material was charged in a Claisen flask having a side arm and an inner capacity of about 100 ml and the bottom portion of the flask was wrapped with a hood on an electric heater. Heating was carried out under a reduced pressure of 0.665 kPa (5 mmHg) and the inner temperature of the flask was maintained at 240°C for 1 hour. After natural cooling, the sublimation product adhering to the inner wall of the upper portion and side arm of the flask was collected, and 35 ml of water was added to the collected sublimation product and the mixture was stirred at room temperature for a while. The insoluble substance (crude VT) was collected by filtration and was recrystallized from 110 ml of water to obtain 11 g (the yield being 80%) of intended VT characterized by a melting point of 239 to 241°C and a TLC value (silica and EtOH, coloration with $I_2$) of Rf = 0.00 to 0.01. The filtrate was distilled under reduced pressure to recover 5.8 g of 2-methylimidazole having a melting point of 115 to 118°C (the recovery ratio was 71%).

Example 3

In 7 ml of water were dissolved 24.7 g (0.1 mole) of a crystal of 2-(β-2'-ethyl-4'(5')-methylimidazolyl-1'))-ethyl-4,6-diamino-S-triazine and 4.8 g (0.02 mole) of $Na_2S \cdot 9H_2O$, and the aqueous solution was sufficiently mixed, and dried and solidified under reduced pressure to obtain a starting material. The starting material was charged in a Claisen flask having a side arm and an inner capacity of about 100 ml. The bottom portion of the flask was wrapped with a hood on an electric heater and heating was carried out under a reduced pressure of 0.665 kPa (5 mmHg) to maintain the inner temperature of the flask at 300°C for 1 hour. After natural cooling the distillation product adhering to the upper portion and side arm of the flask was collected and washed with 35 ml of methanol at room temperature, and the insoluble substance (crude VT) was recovered by filtration in an amount of 9.6 g (0.07 mole) (the yield being 70%). The melting point of the recovered insoluble substance was 235 to 240°C. The insoluble substance was recrystallized from water to obtain 8.9 g (0.065 mole) (the yield being 65%) of purified intended VT characterized by a melting point of 239 to 241°C and a TLC value (silica and EtOH, coloration with $I_2$) of Rf – 0.00 to 0.01. The filtrate was distilled under reduced pressure to recover 9.35 g of 2-methyl-4(5)-methylimidazole (0.085 mole, the yield being 85%).

Example 4

In 12 ml of water were dissolved 34.8 g (0.1 mole) of a crystal of 2-(β-(2'-methylimidazolyl-1'))-ethyl-4,6-diamino-S-triazine/isocyanuric acid adduct and 1.2 g (0.005 mole) of $Na_2S \cdot 9H_2O$, and the aqueous solution was sufficiently stirred, and dried and solidified under reduced pressure to obtain a starting material. The starting material was charged in a Claisen flask having a side arm and an inner capacity of about 100 ml, and the bottom portion of the flask was wrapped with a hood on an electric heater. Heating was carried out under a reduced pressure of 0.665 kPa (5 mmHg) and the inner temperature of the flask was maintained at 240°C for 1 hour. After natural cooling, the sublimation product adhering to the inner wall of the upper portion and side arm of the flask was collected. Then, 50 ml of a 0.2N NaOH aqueous solution was added to the collected sublimation product and the mixture was heated and stirred for a while. The insoluble (crude VT) was recovered by filtration and recrsytallized from 100 ml of water to obtain 10.4 g (the yield being 76%) of intended VT characterized by a melting point of 239 to 241°C and a TLC value (silica and EtOH, coloration with $I_2$) of Rf = 0.00 to 0.01.

Example 5

In 7 ml of water were dissolved 21.9 g (0.1 mole) of 2-(β-(2'-methylimidazolyl-1'))-ethyl-4,6-diamino-S-triazine and 2.2 g (0.008 mole) of $Na_2S \cdot 9H_2O$, and the aqueous solution was sufficiently mixed, and dried and solidified under reduced pressure to form a starting material. The starting material and 25 g of alumina powder were charged in a Claisen flask having a side arm and an inner capacity of about 100 ml, and the bottom portion of the flask was wrapped with a hood on an electric heater. Heating was carried out under a reduced pressure of 0.665 kPa (5 mmHg) and the inner temperature of the flask was maintained at 320°C for 45 minutes. After natural cooling, the sublimation product adhering to the inner wall of the upper portion and side arm of the flask was collected, and 50 ml of a 0.2N NaOH aqueous solution was added to the collected sublimation product and the mixture was heated and stirred for a while. The insoluble substance (crude VT) was recovered by filtration and recrystallized from 100 ml of water to obtain 10.7 g (the yield being 78%) of intended VT characterized by a melting point of 239 to 241°C and a TLC value (silica and EtOH, coloration with $I_2$) of Rf = 0.00 to 0.01. Incidentally, the recovery ratio of 2-methylimidazole was 82%.

**Claims**

1. A process for the synthesis of 2-vinyl-4,6-diamino-S-triazine having the following structural formula:

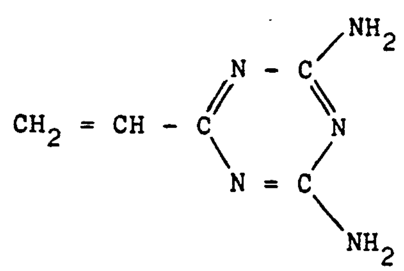

which comprises heating under reduced pressure and in the presence of a polymerization inhibitor, an imidazolyl-S-triazine compound of general formula

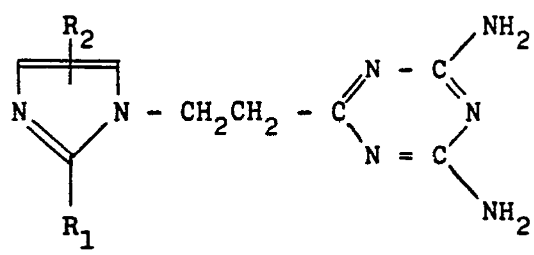

wherein $R_1$ is hydrogen, methyl or ethyl and $R_2$ is hydrogen or methyl, or the isocyanuric acid-addition product thereof.

2. A process according to claim 1, wherein the imidazolyl-S-triazine compound is 2-(β-imidazolyl-(1'))-ethyl-4,6-diamino-S-triazine.

3. A process according to claim 1, wherein the imidazolyl-S-triazine compound is 2-(β-((2'-methyl-imidazolyl-1'))-ethyl-4,6-diamino-S-triazine.

4. A process according to claim 1, wherein the imidazolyl-S-triazine compound is 2-(β-(2'-ethyl-4'(5')-methylimidazolyl-1'))-ethyl-4,6-diamino-S-triazine.

5. A process according to claim 1 or claim 3, wherein the isocyanuric acid-addition product of the

imidazolyl-S-triazine compound is the isocyanuric acid-addition product of 2-(β-(2'-methylimidazolyl-1'))-ethyl-4,6-diamino-S-triazine of the following formula:

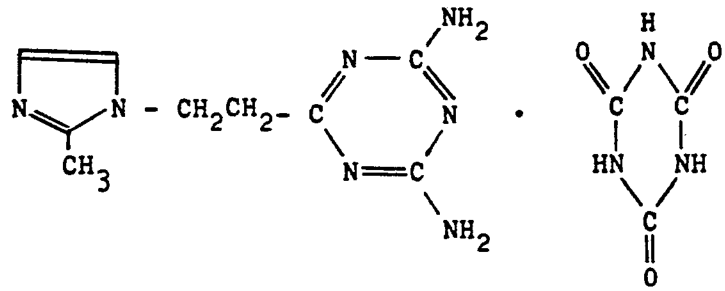

6. A process according to any one of claims 1 to 5, wherein the polymerization inhibitor is sodium sulfide.

7. A process according to any one of claims 1 to 6, wherein heating under reduced pressure is carried out in the co-presence of a powdery or granular heat-conducting medium.

8. A process according to claim 7, wherein the heat-conducting medium is a quartz sand.

9. A process according to claim 7, wherein the heat-conducting medium is alumina powder.

10. A process according to any one of claims 1 to 9, wherein the imidazolyl-S-triazine compound is heated to 180°C to 320°C under a pressure of 0.665 to 2.66 kPa (5 to 20 mmHg).

**Patentansprüche**

1. Vefahren zur Synthese von 4,6-Diamino-2-vinyl-S-triazin mit der nachfolgenden Strukturformel:

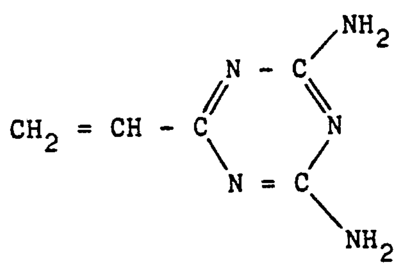

durch Erhitzen unter reduziertem Druck und in Anwesenheit eines Polymerisationsinhibitors von einer Imidazolyl-S-triazin Verbindung der allgemeinen Formel:

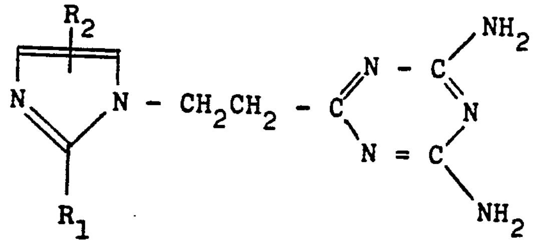

worin $R_1$ für Wasserstoff, Methyl oder Ethyl und $R_2$ für Wasserstoff oder Methyl steht, oder von deren Isocyanursäure-Additionsverbindung.

2. Verfahren nach Anspruch 1, wobei die Imidazolyl-S-triazin Verbindung 2-(β-Imidazolyl-(1'))-ethyl-4,6-diamino-S-triazin ist.

3. Verfahren nach Anspruch 1, wobei die Imidazolyl-S-triazin Verbindung 2-(β-(2'-Methylimidazolyl-1'))-ethyl-4,6-diamino-S-triazin ist.

4. Verfahren nach Anspruch 1, wobei die Imidazolyl-S-triazin Verbindung 2-(β-(2'-Ethyl-4'(5')-methylimidazolyl-1'))-ethyl-4,6-diamino-S-triazin ist.

5. Verfahren nach Anspruch 1 oder Anspruch 3, wobei die Isocyanursäure-Additionsverbindung der

Imidazolyl-S-triazin Verbindung das Isocyanursäure-Additionsprodukt von 2-(β-(2'-Methylimidazolyl-1'))-ethyl-4,6-diamino-S-triazin nachstehender Formel ist:

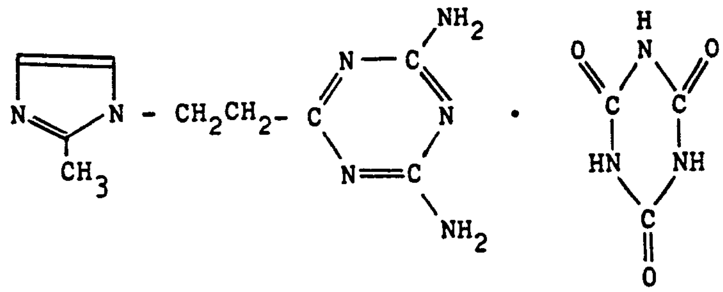

6. Verfahren nach einem der Ansprüche 1 bis 5, wobei der Polymerisationsinhibitor Natriumsulfid ist.

7. Verfahren nach einem der Ansprüche 1 bis 6, wobei das Erhitzen unter reduziertem Druck bei gleichzeitiger Anwesenheit eines pulverförmigen oder granulierten wärmeleitenden Mediums durchgeführt wird.

8. Verfahren nach Anspruch 7, wobei das wärmeleitende Medium Quarzsand ist.

9. Verfahren nach Anspruch 7, wobei das wärmeleitende Medium Aluminiumoxidpulver ist.

10. Verfahren nach einem der Ansprüche 1 bis 9, wobei die Imidazolyl-S-triazin Verbindung auf 180°C bis 320°C unter einem Druck von 0.665 bis 2.66 kPa (5 bis 20 mm Hg) erhitzt wird.

**Revendications**

1. Procédé pour la synthèse de la 2-vinyl-4,6-diamino-S-triazine ayant la formule structurale suivante:

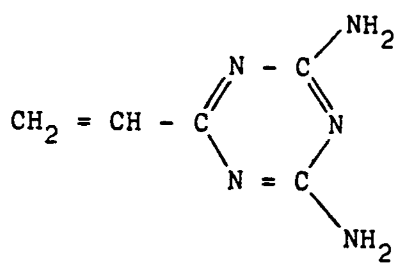

qui consiste à chauffer sous pression réduite et en présence d'un inhibiteur de polymérisation un composé d'imidazolyl-S-triazine de la formule générale:

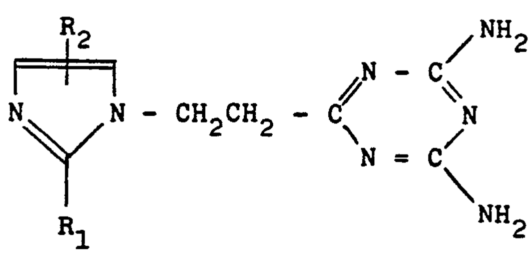

dans laquelle $R_1$ est de l'hydrogène, un groupe méthyle ou un groupe éthyle, et $R_2$ est l'hydrogène ou un groupe méthyle, ou son produit d'addition avec l'acide isocyanurique.

2. Procédé selon la revendication 1, dans lequel le composé d'imidazolyl-S-triazine est 2-(β-imidazolyl-(1'))-éthyl-4,6-diamino-S-triazine.

3. Procédé selon la revendication 1, dans lequel la composé d'imidazolyl-S-triazine est 2-(β-(2'-méthyl-imidazolyl-1'))-éthyl-4,6-diamino-S-triazine.

4. Procédé selon la revendication 1, dans lequel le composé d'imidazolyl-S-triazine est 2-(β-(2'-éthyl-4'(5')-méthylimidazolyl-1'))-éthyl-4,6-diamino-S-triazine.

5. Procédé selon la revendication 1 ou la revendication 3, dans lequel le produit d'addition de l'acide

isocyanurique et du composé d'imidazolyl-S-triazine est le produit d'addition de l'acide isocyanurique et de la 2-(β-(2'-méthylimidazolyl-1'))-éthyl-4,6-diamino-S-triazine de la formule suivante:

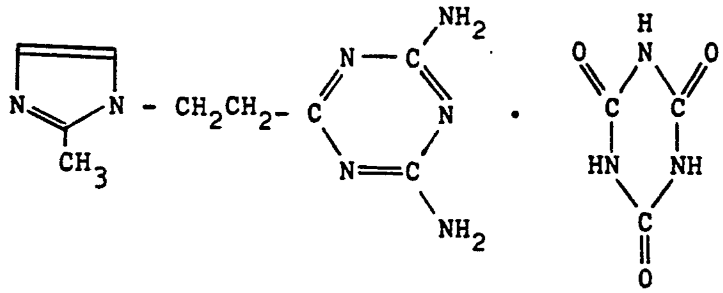

6. Procédé selon l'un quelconque des revendications 1 à 5, dans lequel l'inhibiteur de polymérisation est le sulfure de sodium.

7. Procédé selon l'un quelconque des revendications 1 à 6, dans lequel le chauffage sous pression réduite est effectué en présence d'un produit pulvérulent ou granulaire conducteur de la chaleur.

8. Procédé selon la revendication 7, dans lequel le produit conducteur de la chaleur est du sable de quartz.

9. Procédé selon la revendication 7, dans lequel le produit conducteur de la chaleur est de la poudre d'alumine.

10. Procédé selon l'une des revendications 1 à 9, dans lequel le composé d'imidazolyl-S-triazine est chauffé entre 180°C et 320°C sous une pression de 0,665 à 2,66 kPa (5 à 20 mm Hg).